# DEMANDE DE BREVET EUROPEEN

(11) **EP 1 647 308 A1**
(43) Date de publication de la demande: **19.04.2006**
(21) Numéro de dépôt: 05292145.9
(22) Date de dépôt: 13.10.2005
(51) Int. Cl.: A61Q 3/02, A61Q 5/06, A61K 8/11, A61K 8/40, A61K 8/81

(54) **Utilisation d'une composition à base de monomères électrophiles et de micro- ou nanoparticules**

(30) Priorité: 13.10.2004 FR 0410806
(71) Demandeur: L'OREAL, 75008 Paris (FR)
(72) Inventeur: Brun, Gaelle, 75015 Paris (FR); Livoreil, Aude, 75006 Paris (FR); Gourlaouen, Luc, 92600 Asnieres (FR); Vic, Gabin, 60280 Venette (FR); Giroud, Franck, 92110 Clichy (FR); Rollat-Corvol, Isabelle, 75017 Paris (FR)
(74) Mandataire: Casalonga, Axel

(57) **Abrégé**

La présente demande a pour objet l'utilisation pour le traitement des matières kératiniques, et notamment des fibres kératiniques telles que les cheveux, d'une composition comprenant, dans un milieu cosmétiquement acceptable, au moins un monomère électrophile et des micro- ou nanoparticules.

## Description

La présente invention concerne l'utilisation de compositions à base de monomères polymérisables in situ, d'un milieu cosmétiquement acceptable, et de micro- ou nanoparticules, pour le traitement cosmétique des matières kératiniques, et en particulier des fibres kératiniques telles que les cheveux, ainsi qu'à de nouvelles compositions pour le traitement cosmétique des matières kératiniques.

Par "matières kératiniques", on entend de préférence les fibres kératiniques, et encore de préférence les cheveux.

Il existe de nombreux produits de coiffage permettant d'apporter du volume aux cheveux. Un inconvénient lié à ces produits, le plus souvent à base de polymères filmogènes, réside dans le fait que l'effet cosmétique disparaît dès le premier shampooing.

On connaît par ailleurs des traitements permanents des fibres kératiniques. Ces traitements utilisent un agent réducteur et un agent oxydant, et nécessitent une mise sous tension mécanique des cheveux à l'aide d'un matériel d'enroulage, afin de conférer une mise en forme

Ces procédés, qui permettent effectivement d'augmenter le volume de la chevelure, présentent toutefois l'inconvénient de modifier le niveau de frisure de la chevelure et de dégrader le toucher de la fibre.

Il apparaît ainsi nécessaire de développer des compositions permettant d'accroître le volume de la chevelure sans modifier la forme ou le toucher des cheveux, le tout étant rémanent aux shampooings.

La présente invention propose de nouvelles compositions, permettant de remédier à ces inconvénients.

La demanderesse a découvert, de manière surprenante, qu'en mettant en oeuvre des monomères électrophiles tels qu'ils sont décrits dans la demande de brevet FR2840208 et des micro- ou nanoparticules, il était possible d'apporter du volume à la coiffure, sans altération du toucher des cheveux, ni de leur forme, sans dégradation de la fibre et sans adhésion des cheveux entre eux. En outre, ces propriétés cosmétiques sont rémanentes à plusieurs shampooings.

En plus du volume certaines particules permettent également d'apporter de manière rémanente à la chevelure de la brillance, du corps, de la masse, des effets optiques.

La demanderesse a également constaté qu'en appliquant une composition à base de tels monomères et de micro- ou nanoparticules sur la chevelure, il se formait in situ un revêtement rémanent enrobant les objets.

L'invention a donc pour objet l'utilisation pour le traitement des matières kératiniques, et notamment des fibres kératiniques telles que les cheveux, d'une composition comprenant, dans un milieu cosmétiquement acceptable, au moins un monomère électrophile, et des micro- ou nanoparticules.

Elle a également pour objet un procédé de traitement cosmétique des matières kératiniques, et notamment des fibres kératiniques telles que les cheveux, mettant en oeuvre cette composition.

Elle a également pour objet une composition cosmétique comprenant, dans un milieu cosmétiquement acceptable, au moins un monomère électrophile, et des micro- ou nanoparticules autres que les particules constituées exclusivement d'or ou d'argent.

Pour ces nouvelles compositions, si les micro- ou nanoparticules sont exclusivement métalliques, elles sont de préférence choisies parmi l'aluminium, le cuivre, le fer, le zinc, l'étain, le manganèse, le zirconium. Encore plus préférentiellement, pour ces compositions, les micro- ou nanoparticules ne sont pas exclusivement métalliques.

Elle a enfin pour objet un kit comprenant une première composition contenant au moins un monomère électrophile (présent à des teneurs pouvant être comprise entre 0,5 et 50% du poids de la première composition) et éventuellement au moins un inhibiteur de polymérisation anionique et/ou radicalaire (présent à des teneurs pouvant être comprise entre 10 ppm et 5% du poids de la première composition), ainsi qu'une deuxième composition comprenant dans un milieu cosmétiquement acceptable des micro- ou nanoparticules (présentes à des teneurs pouvant être comprise entre 0,001 et 5% du poids de la deuxième composition).

D'autres objets de l'invention apparaîtront à la lecture de la description et des exemples qui suivent.

On entend par nanoparticule, toute particule dont la taille élémentaire est comprise entre 1nm et 999 nm et par microparticule toute particule dont la taille élémentaire est comprise entre 1µm et 300µm.

Cette nano- ou microparticule peut être sous forme d'une sphère, d'aiguilles, de flocons, de plaquettes, de tube, de fibre, de cube, de prisme ou avoir une forme irrégulière.

Par taille des particules, on entend la distance entre les deux points les plus éloignés de la particule.

On peut citer comme nano- ou microparticule à titre non limitatif les nanoparticules semiconductrices électroluminescentes (ou Quantum Dots), les nano ou microfibrilles, les microplaquettes, les latex, les nanotubes, les microobjets adhésifs, les particules expansibles.

Les particules peuvent être minérales, organiques ou mixtes.

Les particules peuvent donc être minérales. Ces dernières peuvent être en particulier :
- des particules métalliques :
   On entend par particules métalliques des particules formées exclusivement par des métaux choisis parmi les métaux alcalinoterreux, les métaux de transition, les métaux des terres rares et des alliages de ces métaux. On préfère utiliser en particulier l'aluminium, le cuivre, le cadmium, le sélénium, l'argent, l'or, l'indium, le fer, le platine, le nickel, le molybdène, le silicium, le titane, le tungstène, l'antimoine, le palladium, le zinc, l'étain et les alliages de ces métaux, et parmi ceux-ci tout particulièrement l'or, l'argent, le palladium, le platine, le cadmium, le sélénium et les alliages de ces métaux. Ces particules métalliques peuvent être des nanoparticules métalliques organomodifiées portant à leur surface une monocouche autoassemblée de composés organosoufrés telles que décrites dans la demande de brevet FR 2838052.
- des oxydes. On peut citer les oxydes de titane, zinc, cérium, zirconium, aluminium, oxychlorure de bismuth
- des carbures, nitrures, borures, sulfures et hydroxydes.
- des sels inorganiques. On peut citer le sulfate de baryum, carbonate de calcium, sulfate de calcium, phosphate de calcium, hydrocarbonate de magnésium.

Parmi les particules minérales appartenant aux espèces décrites ci-dessus, on peut aussi citer les argiles, les silicates, l'alumine, la silice, le kaolin, hydroxyapatite.

Les particules peuvent être aussi organiques:

Lorsque la particule est de nature organique, il s'agit généralement d'un polymère organique. Ce polymère doit être à l'état vitreux, c'est-à-dire avoir une température de transition vitreuse significativement plus élevée que la température ambiante ou la température d'utilisation (par exemple la température du corps humain), et/ou doit être réticulé.

La température de transition vitreuse des polymères organiques utilisables pour la phase solide est de préférence supérieure à 40 °C, de préférence supérieure à 60°C et en particulier comprise entre 80°C et 200 °C.

Parmi ces polymères, on peut citer de façon non-exhaustive, le polystyrène, le poly(acétate de vinyle), le poly(α-méthylstyrène), le poly(acrylamide), le poly(acrylonitrile), le poly(chlorure de vinyle), les copolymères à base de styrène et de (méth)acrylate d'alkyle en C₁₋C₄, les copolymères à base de styrène et d'acrylamide, les copolymères à base de styrène et d'acrylonitrile, les copolymères à base de styrène et d'acétate de vinyle, les copolymères à base d'acrylamide et de (méth)acrylates d'alkyle en C₁-C₄, les copolymères à base d'acrylonitrile et de (méth)acrylate d'alkyle en C₁-C₄, les copolymères à base d'acrylonitrile et d'acrylamide, les terpolymères à base de styrène, d'acrylonitrile et d'acrylamide, le poly(méthacrylate de méthyle), le poly(méthacrylate d'éthyle), les copolymères styrène/butadiène, styrène/acide acrylique, styrène/vinylpyrrolidone et butadiène/acrylonitrile.

A titre non limitatif on peut citer comme micro- ou nanoparticules organiques :
- les poudres de Nylon, par exemple celle commercialisée sous la dénomination "ORGASOL 2002 ED NAT COS" par la Société ATOCHEM,
- les poudres de polyéthylène, par exemple celle commercialisée sous la dénomination "COATHYLENE HA 1681" par la Société PLAST LABOR,
- les poudres de poly-β-alanine,
- les poudres polyfluorées notamment de polytétrafluoroéthylène, par exemple celle commercialisée sous la dénomination "MP 1400" par la Société DUPONT DE NEMOURS,
- les poudres de copolymère acrylique, telles que celles commercialisées sous la dénomination "POLYTRAP Q5 6603" par la Société DOW CHEMICA,
- les poudres de polystyrène telles que celles commercialisées sous la dénomination "POLYSPHERE 3 000 SP" par la Société PRESPERESE
- les poudres de polyester,
- les microsphères expansées en matériau thermoplastique, par exemple celle commercialisée sous la dénomination "EXPANCEL 551 DE" par la Société EXPANCEL,
- les microbilles de résine de silicone (Tospearls de la Société TOSHIBA, par exemple),
- les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium,
- les poudres de polymères hydrophiles, qui sont d'origine synthétique comme les polyacrylates, par exemple celui commercialisé sous la dénomination "MICROPEARL M 100" par la Société MATSUMOTO,
- les polyamides acryliques, tels que ceux commercialisés par la Société ORIS,
- les polyuréthanes insolubles, tels que celui commercialisé sous la dénomination "PLASTIC POWDER D 800" par la Société TOSHNU
- les microsphères poreuses de cellulose
- les micro- ou nanoparticules de PTFE (polytétrafluoroéthylène).

Les particules peuvent être traitées par enrobage ou greffage. On peut ainsi notamment obtenir des particules mixtes minéral/organique.

Les particules peuvent également être des composés qui ont été rendus hydrophiles par enrobage ou greffage chimique à l'aide de matières telles que le chitosane, le dioxyde de titane, la silice ou des polymères hydrophiles, notamment des polyesters sulfoniques ou des polyammonium quaternaire.

On peut également choisir des composés pulvérulents hydrophobes, choisis parmi des composés pulvérulents de nature aussi bien hydrophobe qu'hydrophile. Dans ce dernier cas, ils sont rendus hydrophobes par enrobage ou greffage chimique par des produits tels que les silicones, les aminoacides, les savons métalliques, les dérivés fluorés, les huiles minérales, la lécithine, le triisostéaroyl titanate d'isopropyle, le polyéthylène, le collagène et ses dérivés, les pol yacryl ates.

A titre d'exemple, on peut citer les microbilles de silice enrobées de polymethylhydrogenosiloxane vendues sous la dénomination commerciale "Silice SI SB 700" par Miyoshi ou encore la sericite enrobée de methicone/huile d'oeuf hydrogénée vendue sous la dénomination commerciale "Sericite SNI S100" par Miyoshi.

Les particules peuvent être choisies parmi les particules adhésives.

Le polymère adhésif peut être immobilisé à la surface de la particule par des liaisons chimiques covalentes (greffage) ou par des interactions physico-chimiques faibles telles que l'interaction hydrophobe, les liaisons hydrogène et les forces de van der Waals (adsorption).

Le caractère adhésif d'un polymère organique est généralement lié à température de transition vitreuse de celui-ci. Une condition nécessaire mais non suffisante pour qu'un polymère soit adhésif est une température de transition vitreuse (Tg) significativement inférieure à la température ambiante ou à la température d'utilisation. Les polymères organiques adhésifs utilisés pour la préparation des micro-objets de la présente invention ont de préférence une température de transition vitreuse inférieure ou égale à 10 °C, de préférence inférieure ou égale à 0 °C.

La nature chimique des polymères organiques adhésifs n'est pas déterminante pour la présente invention pour autant que le dépôt de polymère présente les caractéristiques d'adhésivité et/ou d'auto-adhésivité ci-dessus. Ces polymères adhésifs pourront être réticulés ou non. Pour trouver des exemples concrets de polymères adhésifs, on pourra se référer aux demandes suivantes décrivant des particules ou des polymères adhésifs :

WO98/38969, FR 2833960 (Polyuréthanes cationiques ou amphotères auto-adhésifs), FR 2833959 (Polymères radicalaires cationiques ou amphotères autoadhésifs).

Les particules peuvent également être choisies parmi les nanotubes.

Les nanotubes peuvent être constitués d'au moins un élément appartenant aux groupes IIA, IIIA, IVA, VA, VIII, IB, IIB, IIIB, VIB et VIIB de la classification périodique.

On entend par nanotubes des nano-objets dont l'organisation des atomes ou des molécules confère à la nano-structure une forme de tube. Ces nanotubes peuvent être à simple paroi ou multi-parois. Le diamètre des nanotubes est classiquement compris entre 1 et 300 nm et la longueur des nanotubes est classiquement comprise entre 10 nm et 10 mm.

A titre d'exemples d'éléments constitutifs des nanotubes de l'invention, on peut citer le carbone, le silicium, le tungstène, l'argent, l'or, le bore, le zinc, le platine, le magnésium, le fer, le cérium et l'aluminium.

De préférence, les nanotubes sont constitués d'au moins un élément appartenant au groupe IVA, de la classification périodique des éléments. Encore plus préférentiellement, ledit élément est le carbone.

Lorsqu'un des éléments constitutifs des nanotubes est le carbone, lesdits nanotubes peuvent être constitués totalement ou partiellement de molécules organiques. A titre d'exemple de molécules organiques on peut citer les phospholipides diacétyléniques, les glutamates, les diamides à longue chaîne, les glucophospholipides, les alkylphénylglucopyranosides.

Préférentiellement, le squelette des nanotubes est constitué uniquement d'atomes de carbone.

Ces nano-formes carbonées sont classiquement obtenues par sublimation de graphite à très haute température par l'intermédiaire d'un arc électrique. Les nanotubes de carbone peuvent être formés par un seul plan de graphène ; dans ce cas ils sont dits à simple paroi (Single Wall NanoTube SWNT en anglais). L'enroulement des plans de graphène peut être en zig-zag, créneau ou chiral. Les nanotubes peuvent être également constitués par plusieurs tubes « emboîtés » les uns dans les autres ; dans ce cas ils sont dits nanotubes multi-parois (Multi Wall NanoTube MWNT en anglais).

Selon un mode de réalisation de l'invention, afin d'obtenir une solubilisation ou une exfoliation optimale des nanotubes de carbone dans le milieu cosmétique considéré, la surface des nanotubes est fonctionnalisée.

Par l'expression « fonctionnalisée », on entend selon l'invention la présence de groupements fonctionnels pouvant interagir physiquement ou chimiquement entre eux ou avec le milieu extérieur.

Tous mécanismes réactionnels peuvent être utilisés pour fonctionnaliser les plans de graphène constituant les nanotubes de carbone. A titre d'exemple, la fonctionnalisation des nanotubes de carbone peut être réalisée par un mécanisme réactionnel du type substitution nucléophile, substitution électrophile, substitution radicalaire, addition, élimination, réarrangement, oxydation, réduction, réaction acido-basique, réaction électrochimique, ou encore réaction photochimique.

Parmi les fonctions pouvant être greffées à la surface des plans de graphène constituant le nanotube de carbone, on peut citer les groupements carboxyliques. Cette fonctionnalisation est décrite dans l'article *« Solution Properties of Single Walled Carbone Nanotube », J . Chen et Coll (Science 1998, vol 282, no 5396, pages 95-98).*

On peut également citer la solubilisation des nanotubes dans un solvant polaire tel que eau, éthanol, par oxydation des plans de graphène par un mélange HCl/CrO3, décrite dans l'article « *Room Temperature Filling of Single Wall Carbon Nanotubes With Oxide inopen air », J . Mittal et Coll. (Chem. Phys. Lett. 2001, vol 339, n "5-6, pages 311-318)* ou encore par condensation sur les nanotubes d'un acide aminé et d'un aldéhyde *(J . Am. Soc., vol 124, n"5, 2002, pages 760 et 761).*

Des fonctions hydrophobes peuvent également être greffées à la surface des plans de graphène constituant le nanotube de carbone. On peut par exemple citer la fluoration des nanotubes de carbone décrite dans l'article *« Fluorinated Single Wall nanotubes », K.N. Kudin et Coll. (Phys. Rev. B63, 45413).*

On peut également greffer des molécules inorganiques telles que les alcoxysilanes *(Nano. Lett., vol 2, n"4, 2002 pages 329 à 332).*

La fonctionnalisation des plans de graphène peut s'effectuer en plusieurs étapes, comme par exemple la fonctionnalisation des nanotubes de carbone par des amides à chaîne grasse, décrite dans l'article *« Dissolution of Single Wall Carbon Nanotube », M. A. Hamon et coll. (Adv. Mater. 1999, 11, n°10).* Cette fonctionnalisation en plusieurs étapes peut également conduire au greffage du glucose *(Nano. Lett., vol 2, n°4, 2002 pages 369 à 373).*

La fonctionnalisation des nanotubes de carbone peut être réalisée par de simples molécules, mais également par des oligomères, des polymères ou des dendrimères. On peut citer à titre d'exemple l'article *«A New Purification Method for Single Wall Carbon Nanotubes », M. Holzinger et Coll (Appl. Phys. A 70 (2000) 599),* qui décrit le greffage de structures dendritiques à la surface des plans de graphène constituant le nanotube de carbone.

Outre l'amélioration de la dispersion des nanotubes de carbone dans les milieux cosmétiques, la fonctionnalisation de la surface peut également être réalisée afin d'augmenter l'affinité des nanostructures carbonées pour la matière kératinique. L'amélioration de l'affinité entre les nanotubes et la matière kératinique induite par la fonctionnalisation des plans de graphène peut être le fruit de l'accroissement des interactions de type Van der Waals et/ou le fruit de l'apparition de liaions hydrogène et/ou ionique. Ainsi, le ou les groupements fonctionnels sont susceptibles de créer avec les fibres kératiniques une ou plusieurs liaisons chimiques choisies parmi les interactions de type Van der Waals, les liaisons hydrogène, les liaisons ioniques et les liaisons covalentes. Dans ce cadre on peut citer le greffage de molécules cationiques à la surface de nanotubes de carbone, décrit dans l'article *« Exohedral Sidewall Reactions of Single Walled Carbon Nanotubes in Molecular Nanostructures », M. Holzinger et all (Proceeding of the XIIth International Winterschool on Electronic Properties of Novel Materials :Molecular Nanostructures, Kirchberg, Austria, March 2001).* Dans le cadre de greffage de molécules cationiques on peut aussi mentionner le greffage de dérivés de polyéthylèneimine *(Nano. Lett., vol 2, n "3, 2002 pages 231 à 234).*

Les nanotubes peuvent aussi être polymériques.

Le polymère utilisé pour obtenir le nano-objet est un polymère synthétique. Par « polymère synthétique » au sens de la présente demande, on entend un polymère obtenu par synthèse chimique ou électrochimique (polymérisation radicalaire, polycondensation, polymérisation par ouverture de cycle, polymérisation par métathèse).

La réticulation peut se faire par voie chimique ou sous l'action du rayonnement photochimique comme par exemple sous l'action des UV ou de la température. Ce polymère peut-être un homopolymère ou un copolymère.

Conviennent tout particulièrement à l'invention, les homopolymères ou copolymères dérivant de la polymérisation radicalaire de monomères comprenant des motifs éthyléniques, vinyliques, allyliques, (méth)acrylates et/ou de (méth)acrylamides et dérivés. Ainsi conviennent les copolymères vinyliques/(méth)acrylates, vinyliques/(méth)acrylamides, vinyliques/(méth)acrylates/ (méth)acrylamides, oléfiniques/vinyliques et les (méth)acrylates/ (méth)acrylamides.

Ces nanotubes de polymères sont notamment décrits dans les publications suivantes :
- Nanotube formation from renewable resources via coiled nanofibers, G. John, M. Masuda, Y. Okada, K. Yase, T. Shimizu. Advanced Materials, 2001, 13, 715-718
- Bottom-up synthesis ans structural properties of self-assembled high-axial-ratio nanostructures, T; Shimizu, Macromol. Rapid Commun., 2002, 23, 311-331

Les particules peuvent aussi être choisies parmi les nanoparticules semiconductrices.

Les nanoparticules semiconductrices luminescentes sont capables d'émettre sous l'action d'une excitation lumineuse, un rayonnement présentant une longueur d'onde comprise entre 400 nm et 700 nm dans le domaine de la cosmétique.

Ces nanoparticules présentent la particularité de présenter des spectres d'émission de la couleur beaucoup plus étroits que la plupart des colorants ou pigments organiques utilisés en coloration capillaire. On obtient ainsi des colorations plus pures.

Elle permettent d'obtenir des effets optiques rémanents.

Au sens de la présente invention, le terme effet optique recouvre des effets de brillance, de couleur, métallique, goniochromatique, moiré.

Par ailleurs, et plus particulièrement, il est à noter que la brillance correspond à l'intensité lumineuse réfléchie sous un angle α lorsque la mèche de cheveux est éclairée sous un angle -α. L'angle α classiquement utilisé pour mesurer cette réflexion spéculaire, autrement dit la brillance, est égal à 20°. Cet apport de brillance peut être mesuré par utilisation d'un brillancemètre comme il est par exemple décrit dans la norme ISO 2813 -1994 de l'AFNOR (août 1994. rectificatif février 1997).

De plus, la couleur émise par ces nanoparticules varie en fonction du diamètre de celles-ci. Ainsi, on peut obtenir des gammes de couleurs très variées en utilisant dans les compositions une ou plusieurs tailles de nanoparticules. Ces nanoparticules possèdent aussi la particularité d'émettre des couleurs très intenses.

Selon un mode de réalisation particulier de l'invention, les nanoparticules comprennent au moins un métal choisi parmi Zn, Cd, Hg et au moins un métal choisi parmi S, Se et Te. De préférence, ces nanoparticules comprennent du séléniure de cadmium ou du sulfure de cadmium.

Dans les nanoparticules, les métaux présents peuvent être répartis de façon homogène. Les nanoparticules peuvent aussi être constituées d'un coeur constitué d'un ou plusieurs métaux et d'une ou plusieurs couches recouvrant ce coeur, constituées d'un ou plusieurs métaux différents de ceux constituant le coeur. Ces nanoparticules sont connues dans la littérature sous la forme de nanoparticules core/shell.

Selon ce mode de réalisation particulier, les nanoparticules peuvent présenter un coeur en sélénure de cadmium recouvert d'une couche de sulfure de zinc.

Les nanoparticules peuvent aussi être recouvertes d'une ou plusieurs couches additionnelles organiques et/ou inorganiques, présentant de préférence une affinité pour les cheveux. A titre d'exemple de couche organique, on peut citer les couches obtenues à partir de polyéthylène glycol, polyuréthane, de dextran, de polyacrylique, de polyvinylpyrolidone, de polyvinylcaprolactone.

A titre de couche inorganique, on peut citer à titre d'exemple, les couches obtenues à partir d'alumine, de silice ou d'argile ou un mélange de ces matériaux.

Ces couches peuvent être obtenues par procédé sol-gel à partir d'organosilane. Ces couches qui sont obtenues par encapsulation des nanoparticules peuvent être réalisées par divers procédés tels que la précipitation contrôlée, la séparation de phase, la polymérisation en émulsion, la polycondensation interfaciale, la polycondensation in situ.

Pour plus de détails, de tels procédés d'encapsulation sont décrits dans « Microencapsulation Methods and industrial Applications » (ISBN 0-8247-9703-5)

La capsule peut être formée par tous composés inorganiques, plus spécifiquement par un oxyde métallique ou un polymère organométallique et encore plus spécifiquement par un oxyde métallique ou un polymère organométallique obtenus par procédé sol gel tels que les oxydes métalliques ou les polymères organométalliques synthétisés par polycondensation d'un seul ou d'un mélange d'alcoxy simple ou mixte de silicium, d'aluminium, de bore, de lithium, de magnésium, de sodium, de titane et/ou de zirconium. Pour plus de détails sur la nature des précurseurs et les mécanismes réactionnels, on peut se référer à l'ouvrage « Sol Gel Science» publié par C.J Brinker et G.W. Scherer aux éditions Academic Press (ISBN 0-12-134970-5).

Ces couches additionnelles peuvent être greffées de façon covalente ou être adsorbées à la surface de la nanoparticules.

Selon un mode de réalisation différent, les nanoparticules peuvent être incorporées dans des microbilles de polymère, le polymère pouvant être hydrophile, hydrophobe, amphiphile, ionique ou non-ionique. A titre de polymère, on peut citer les polystyrènes selon les procédés décrits dans : « Quantum-dot-tagged microbeads for multiplexed optical coding of biomolecules », Mingyong Han, Nature Biotechnology Vol. 19, PP. 631-635 July 2001.

Les nanoparticules peuvent présenter des tailles variant de 1 à 100 nm, de préférence entre 1 et 50 nm. De façon particulièrement préférée, ces nanoparticules présentent un diamètre compris entre 1 et 20 nm.

Les nanoparticules sont connues de la littérature. En particulier, ces nanoparticules peuvent être fabriquées selon les procédés décrits par exemple dans le brevet US 6 225 198 ou US 5 990 479, dans les publications qui y sont citées, ainsi que dans les publications suivantes : Dabboussi B.O. et al *"(CdSe)ZnS core-shell quantum dots : synthesis and characterisation of a size series of highly luminescent nanocristallites" Journal of physical chemistry B, vol 101, 1997, pp 9463-9475.* et Peng, Xiaogang et al, *"Epitaxial Growth of highly Luminescent Cdse*/*CdS core*/*shell nanocrystals with photostability and electronic accessibility" Journal of the American Chemical Society, vol 119, N°30, pp 7019-7029.* Les nanoparticules utiles dans la présente invention sont aussi connues sous le nom de "Quantum dots".

A titre d'exemple, on peut citer les nanoparticules suivantes :

| Type de nanoparticules | Taille | Couleurs | Concentration de la solution |
|---|---|---|---|
| CdSe | 2,2nm | Vert | 0,5mg/ml |
| CdSe | 3,4nm | Jaune | 0,5mg/ml |
| CdSe | 4,0nm | Orange | 0,5mg/ml |
| CdSe | 4,7nm | Rouge orangé | 0,5mg/ml |
| CdSe | 5,6nm | Rouge | 0,5mg/ml |
| CdSe/ZnS | 4,3nm | Vert | 0,5mg/ml |
| CdSe/ZnS | 4,8nm | Jaune vert | 0,5mg/ml |
| CdSe/ZnS | 5,4nm | Jaune | 0,5mg/ml |
| CdSe/ZnS | 6,3nm | Orange | 0,5mg/ml |
| CdSe/ZnS | 7,2nm | Rouge | 0,5mg/ml |

Ces nanoparticules sont fournies par la société EVIDENT Technologies.

Les nanoparticules CdSe sont des nanoparticules uniformes qui contiennent uniquement de CdSe. Les nanoparticules CdSe/ZnS ont des structures core/shell avec un « core » de CdSe et une « shell » de ZnS.

Les nano ou micro-particules peuvent également être choisies parmi les nano- ou microfibrilles

On entend par nano ou microfibrilles, des particules telles qu'elles peuvent être décrites dans les publications suivantes :
- Polymerization in nanometer-sized fibers : Molecular Packing Order and Polymerizability: M. Masuda, T. Hanada, Y. Okada, K. Yase, T. Shimizu, Macromolecules, 2000, 33, 9233-9238
- Organic supramolecula self-assembled materials stabilized by multiple hydrogen bonds, T. Shimizu, Transactions of the Materials Research Society of Japan, 1999, 24, 3, 431-436

Ces fibrilles peuvent être naturelles, telles que la cellulose, les protéines, la soie, ou synthétique, comme le polyamide.

Les micro- ou nanoparticules peuvent également être choisies parmi les particules expansibles, et notamment les composés susceptibles de gonfler sous l'action de la chaleur.

Il peut s'agir notamment d'un composé qui réagit, sous l'action de la chaleur, pour libérer un gaz qui se trouve emprisonné dans la matrice du dépôt.

Le composé susceptible de gonfler à la chaleur peut également se présenter sous la forme de particules thermoexpansibles.

Par l'expression « particules thermoexpansibles », on désigne plus particulièrement des particules susceptibles de se déformer et de s'expanser à la chaleur. Les particules au sens de la présente invention peuvent encore être des particules thermodéformables non expansées. Elles se distinguent à ce titre des particules expansées qui ne sont précisément plus sujettes à une déformation sous l'action de la chaleur, à l'image par exemple des particules de polyvinylidène/acrylonitrile commercialisées sous la dénomination générale d' « Expancel® » par la société AKZO NOBEL sous les références particulières « Expancel® WE » ou « DE ».

Ces particules sont capables de s'expanser sous l'action d'une température généralement supérieure ou égale à 45°C, notamment supérieure ou égale à 50°C, en particulier supérieure ou égale à 60°C, plus particulièrement supérieure ou égale à 70°C. En particulier, il peut s'agir d'une température supérieure ou égale à 80°C, en particulier supérieure ou égale à 85°C, plus particulièrement supérieure ou égale à 90°C et allant jusqu'à 190-200°C.

Avantageusement, ces particules ne sont pas sensibles à la présence d'eau.

En particulier les particules peuvent être thermoplastiques. Par l'expression thermoplastique, on désigne des particules qui sont susceptibles de se déformer sous l'action de la chaleur et de conserver leur nouvelle forme, y compris après refroidissement, notamment à température ambiante.

Les particules sont généralement des particules creuses comportant une enveloppe continue et au moins une cavité.

L'enveloppe des particules est de préférence flexible pour se prêter à une déformation mécanique. Elle comprend généralement au moins un polymère, homo- ou copolymère, formé à partir de monomères à insaturations éthyléniques. Des exemples de telles particules sont notamment décrits dans les documents EP-A-56219, EP-A-348 372, EP-A-486 080, EP-A-320 473, EP-A-112 807 et US-A-3 615 972.

Les monomères utilisés peuvent être en particulier des esters d'acide méthacrylique ou acrylique, tels que l'acrylate et le méthacrylate de méthyle, le chlorure de vinylidène, l'acrylonitrile, le styrène et ses dérivés, le butadiène et ses dérivés, et leurs mélanges.

A titre représentatif et non limitatif des polymères susceptibles de composer l'enveloppe des particules, on peut notamment citer des polymères comportant au moins des motifs dérivés d'acrylate ou de méthacrylate de méthyle, des polymères comportant au moins des motifs dérivés d'acrylonitrile, des polymères comportant au moins des motifs dérivés d'acrylonitrile et de méthacrylate de méthyle, des polymères comportant au moins des motifs dérivés de styrène et d'acrylonitrile, des polymères comportant au moins des motifs dérivés de chlorure de vinylidène et d'acrylonitrile et des polymères comportant au moins des motifs dérivés de chlorure de chlorure de vinylidène et de chlorure de vinyle. En particulier, ledit polymère peut être choisi parmi les polymères de chlorure de vinylidène/acrylonitrile/méthacrylate de méthyle, les polymères d'acrylonitrile/méthacrylate de méthyle et les homopolymères d'acrylonitrile.

Les particules contiennent généralement au sein d'une ou plusieurs cavité(s) au moins un composé capable de manifester, en réponse à un chauffage à une température située dans une gamme allant de 45°C à 200°C et à pression sensiblement constante, une augmentation significative de son volume à température ambiante.

On entend par l'expression « augmentation significative de son volume », une augmentation d'au moins un facteur 30, en particulier d'au moins un facteur 40 et plus particulièrement d'au moins un facteur 50 du volume occupé.

De façon générale, le composé contenu à l'intérieur de la cavité peut être à température ambiante un composé gazeux ou bien un composé liquide présentant une température de vaporisation située dans la gamme de 45°C à 200°C, en particulier dans la gamme de 80°C à 200°C, et plus particulièrement supérieure ou égale à 100°C.

Selon une première variante, ce composé est à l'état gazeux dans la particule et se dilate sous l'effet de la chaleur. Parmi les composés à l'état gazeux, on peut citer l'air, l'azote, les hydrocarbures comprenant 1, 2, 3 ou 4 atomes de carbone comme en particulier le butane, l'isobutane et leurs mélanges.

Selon une deuxième variante, le composé contenu dans la cavité est un composé liquide tel que défini précédemment. Parmi ces composés, on peut citer les hydrocarbures, notamment ayant de 5 à 15, en particulier de 5 à 12 et plus particulièrement de 5 à 10 atomes de carbone. Il peut s'agir en particulier de d'un composé, choisi parmi le n-pentane, l'iso-pentane et le néo-pentane.

La température d'expansion de la particule dépend à la fois de la nature du composé présent dans sa cavité, et de celle du polymère formant son enveloppe, et peut en particulier aller de 45 à 200°C, et être notamment supérieure ou égale à 80°C et en particulier supérieure ou égale à 100°C.

Les particules utilisées dans les compositions selon l'invention peuvent être sèches ou hydratées.

Ces particules peuvent être de différentes formes. Elles peuvent être de forme globulaire, voire sphérique, ou peuvent également être allongées.

Selon un mode de réalisation particulier, les particules non expansées de l'invention sont sphériques et présentent une granulométrie, exprimée en diamètre « effectif » moyen en poids D[0,5], allant de 0,5 µm à 200 µm, notamment de 1 µm à 100 µm, en particulier de 4 µm à 50 µm et plus particulièrement de 5 µm à 40 µm.

Selon un mode de réalisation particulier, les particules utilisées dans les compositions selon l'invention ont une forme de fibre. Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150. Les fibres présentent en particulier une longueur allant de 0,05 mm à 6 mm.

Les particules non expansées utilisées dans la présente invention présentent généralement une masse volumique allant de 500 kg/m3 à 5000 kg/m3, en particulier de 900 kg/m3 à 3000 kg/m3, et plus particulièrement de 900 kg/m3 à 2000 kg/m3.

Comme particules utilisables dans les compositions de l'invention, on peut citer par exemple les microsphères non expansées de copolymère de chlorure de vinylidène/d'acrylonitrile/méthacrylate de méthyle comme par exemple celles commercialisées sous la dénomination d'« Expancel® » par la société AKZO NOBEL sous les références 820DU 40 (10-16 µm) ou 820 SL 40 (2-30 µm), et d'acrylonitrile/méthacrylate de méthyle comme par exemple celles commercialisées sous la dénomination d'« Expancel® » sous les références 642 WU 40 (10-16 µm) ou 051 DU 40 (9-15 µm). Comme particules pouvant être également utilisées dans les compositions selon l'invention, on peut encore citer les microsphères non expansées d'homopolymère d'acrylonitrile comme par exemple celles commercialisées sous la dénomination d'« Expancel 007W® » (5-25 µm), de « Micropearl F-series® » par la Société MATSUMOTO ou d'« Ucelite® » par la Société UCB.

Les particules commercialisées sous la dénomination d' « Expancel® » sous les références citées ci-dessus comprennent généralement dans leur cavité un composé à l'état gazeux.

Les micro- ou nanoparticules peuvent être présentes dans la composition à des teneurs comprises entre 0,0001 et 30% en poids, de préférence entre 0,001% et 20%, et encore de préférence entre 0,01% et 10% en poids du poids total de la composition.

Par monomère électrophile, on entend un monomère capable de polymériser par polymérisation anionique en présence d'un agent nucléophile tel que par exemple, les ions hydroxyles (OH-) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
- les dérivés benzylidene malononitrile (A), le 2-(4-chloro-benzylidene)-malononitrile (A1) le 2-cyano-3-phényl acrylate d'éthyle (B), le 2-cyano-3-(4-chloro-phényl) acrylate d'éthyle (B1) décrits dans Sayyah, *J. Polymer Research,* 2000, p97 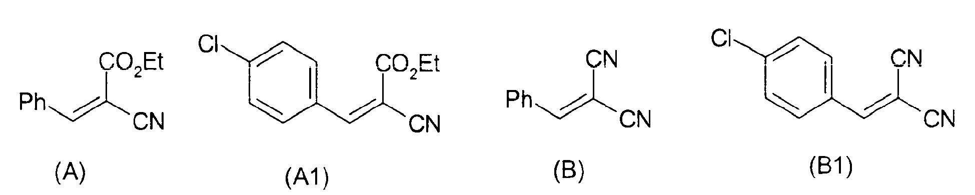
- les dérivés de méthylidenemalonates comme :
   - le 2-méthylene-malonate de diéthyle (C) par Hopff, *Makromoleculare Chemie,* 1961, p95, De Keyser, *J. Pharm. Sci,* 1991, p67 et Klemarczyk, *Polymer,* 1998, p173 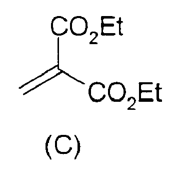
   - le 2-éthoxycarbonylméthyleneoxycarbonyl acrylate d'éthyle (D) par Breton, *Biomaterials,* 1998, p271 et Couvreur, *Pharmaceutical Research,* 1994, p1270. 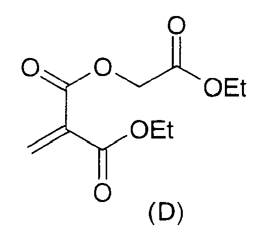
- les dérivés itaconate et itaconimide comme :
   - l'itaconate de diméthyle (E) par Bachrach, *European Polymer Journal,* 1976, p563 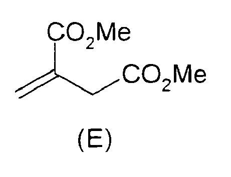
   - N-butyl itaconimide (F), N-(4-tolyl) itaconimide (G), N-(2-ethylphenyl) itaconimide (H), N-(2,6-diethylphenyl) itaconimide (I) par Wanatabe, *J.Polymer Science : Part A :Polymer chemistry,* 1994, p2073 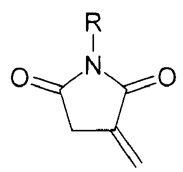 R= Bu (F), 4-tolyl (G), 2-ehylphenyl (H), 2,6-diethyphenyl (I)
- les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O), par Gipstein, *J. Org. Chem.* 1980, p 1486 et
- les dérivés 1,1-bis-(methylsulfonyl)ethylene (P), 1-acetyl-1-methyl sulfonyl ethylene (Q), α-(methylsulfonyl) vinyl sulfonate de methyle (R), α-methylsulfonylacrylonitrile (S) par Shearer, US patent US2748050. 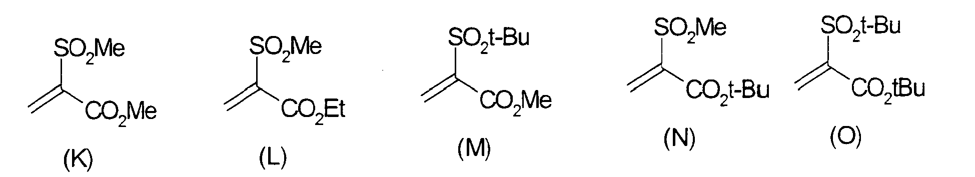 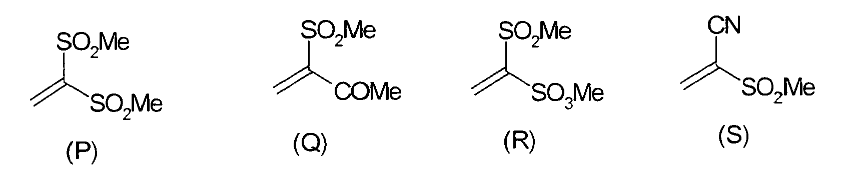
- les dérivés méthyl vinyl sulfone (T) et phényl vinyl sulfone (U) par Boor, *J.Polymer Science,* 1971, p249 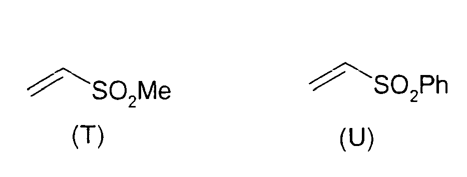
- le dérivé phényl vinyl sulfoxide (V) par Kanga, *Polymer preprints (ACS, Divison of Polymer Chemistry),* 1987, p322 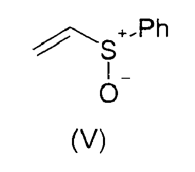
- le dérivé 3-methyl-N-(phenylsulfonyl)-1-aza-1,3-butadiene (W) par Bonner, *Polymer Bulletin,* 1992, p517 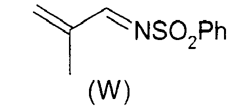
- les dérivés acrylates et acrylamides comme :
   - N-propyl-N-(3-triisopropoxysilylpropyl)acrylamide (X) et N-propyl-N-(3-triethoxysilylpropyl)acrylamide (Y) par Kobayashi, *Journal of Polymer Science, Part A: Polymer Chemistry,* 2005, p2754. 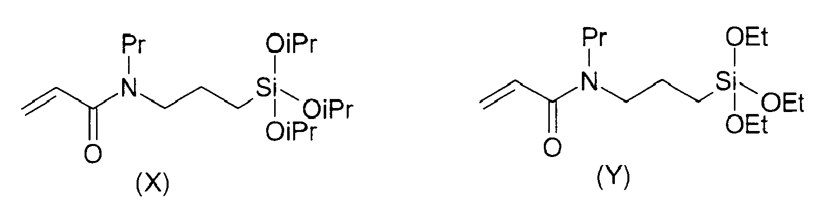
   - 2-hydroxyethyl acrylate (Z) et 2-hydroxyethyl méthacrylate (AA) par Rozenberg, *International Journal of Plastics Technology,* 2003, p17 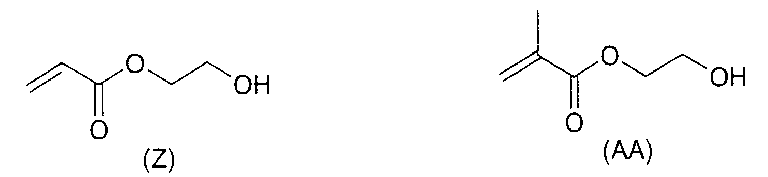
   - N-butyl acrylate (AB) par Schmitt, *Macromolecules,* 2001, p2115
   - Tert-butyl acrylate (AC) par Ishizone, *Macromolecules,* 1999, p955.

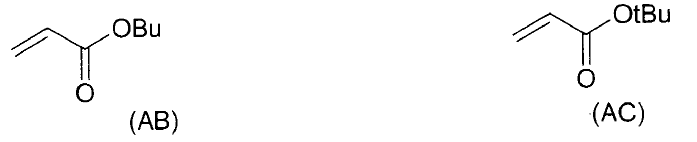

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (1) : 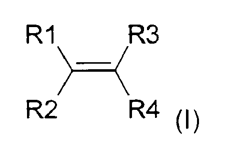 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR,
   - OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, - OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère, R' désignant un radical alkyle en C₁-C₁₀, ce polymère pouvant être obtenu par polymérisation radicalaire, par polycondensation ou par ouverture de cycle.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)n-(CF₂)m-CF₃ ou -(CH₂)n-(CF₂)m-CHF₂ avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoïques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (II) : 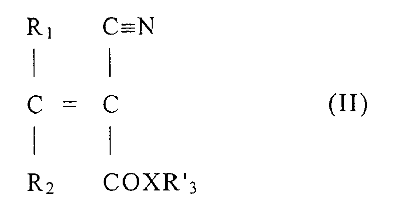 X désignant NH,S,O,
R₁ et R₂ ayant les mêmes significations que précédemment,

R'₃ pouvant désigner un atome d'hydrogène ou un radical R tel que défini pour la formule (A).

De préférence, X désigne O.

A titre de composés de formule (B), on peut citer les monomères :
a) appartenant à la famille des 2-cyanoacrylates de polyfluoroalkyle en C₁-C₂₀ tels que :
   l'ester 2,2,3,3-tétrafluoropropylique de l'acide 2-cyano-2-propénoïque de formule : 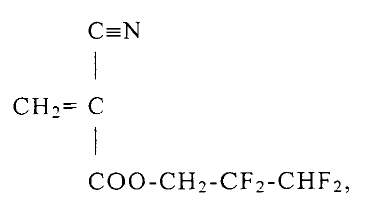
   ou encore l'ester 2,2,2-trifluoroéthylique de l'acide 2-cyano-2-propénoïque de formule : 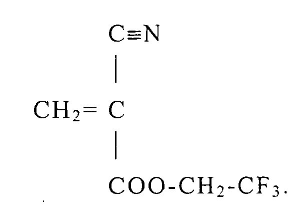
b) les cyanoacrylates d'alkyle en C₁-C₁₀ ou d'(alcoxy en C₁₋C₄)(alkyle en C₁-C₁₀).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères les plus particulièrement préférés sont ceux de formule III et leurs mélanges : 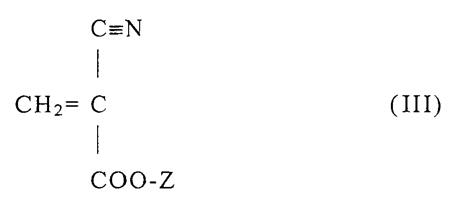 dans laquelle : Z=-(CH₂)₇-CH₃,
-CH(CH₃)-(CH-₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Par milieu cosmétiquement acceptable, on entend un milieu compatible avec les matières kératiniques telles que les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadama, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique, l'alcool béhénylique ; les acides gras en C₁₀-C₃₀ tels que l'acide laurique, l'acide stéarique ; les amides gras en C₁₀-C₃₀ tels que la diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 105 Pa (760mm de Hg).

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, tel que, par exemple, des agents réducteurs, des corps gras, des plastifiants, des adoucissants, des agents anti-mousse, des agents hydratants, des pigments, des filtres UV, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des épaississants minéraux ou organiques tels que le benzylidène sorbitol, les N acylaminoacides. Ces agents peuvent être éventuellement encapsulés. La capsule peut être de type polycyanoacrylate.

Le procédé de traitement des cheveux conforme à l'invention consiste à appliquer la composition décrite ci-dessus sur les matières kératiniques, et en particulier en présence d'un agent nucléophile avec ou sans chauffage.

De préférence, cet agent nucléophile est l'eau. Cette eau peut être apportée par une humidification préalable.

Il est également possible, afin de moduler la cinétique de réaction, de préalablement humidifier les matières kératiniques à l'aide d'une solution aqueuse dont le pH a été ajusté à l'aide d'une base, d'un acide ou d'un mélange acide/base. L'acide et/ou la base peuvent être inorganique ou organique.

Ces deux opérations peuvent aussi être effectuées après application de la composition.

Il est également possible de moduler la cinétique de polymérisation par voie anionique en pré-imprégnant les matières kératiniques à l'aide d'un agent nucléophile. L'agent nucléophile peut être utilisé pur, en solution, sous forme d'une émulsion, ou être encapsulé.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PHNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

De préférence, l'agent nucléophile est l'eau. Cette eau peut être apportée par une humidification préalable. Les agents nucléophiles particulièrement préférés sont les ions hydroxyles, notamment ceux présents dans l'eau.

Il est également possible, afin de moduler la cinétique de réaction, de préalablement humidifier la fibre à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant la fibre à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'invention a également pour objet de nouvelles compositions telles que définies précédemment. Dans ces compositions, les micro- ou nanoparticules peuvent être minérales, organiques ou mixtes, et être enrobées ou greffées. De préférence, ces particules sont des oxydes métalliques, des particules de polymères, des boîtes quantiques, des nanotubes, des nanofibrilles. Tout ce qui a été cité pour les compositions précédentes est également valable pour ces compositions.

L'application des compositions conforme à l'invention peut être suivie ou non d'un rinçage. Ces compositions peuvent se présenter sous des formes diverses, telles que de lotion, de spray, de mousse et être appliquées sous forme de shampooing ou d'après-shampooing.

Le mode d'application peut être en une seule étape ou bien être divisé en étapes successives. Si le procédé comporte plusieurs étapes d'application de compositions actives, ce peuvent être les suivantes :
1. Application sur les cheveux des micro- ou nanoparticules, présentes en solution aqueuse à raison de 0,05 à 40 %, de préférence de 0.1 à 35 % et mieux de 0.25 à 25%
2. Application sur les cheveux humidifiés du monomère, présent en solution à une concentration comprise entre 0,05 et 30 % en poids, plus préférentiellement comprises entre 0,01 et 50 % en poids, et plus préférentiellement entre 0,1 et 20 % en poids.

En plus de l'actif, chaque composition peut contenir des additifs cosmétiques conventionnels. L'ordre des deux premières étapes peut être inversé. La première étape peut être précédée de l'application d'un produit cosmétique. De même, la dernière étape peut être succédée de l'application d'un produit cosmétique. Chaque étape peut être interrompue par un rinçage, un séchage. Le séchage pouvant être effectué au casque, au sèche cheveux et/ou au fer à lisser.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C. ce qui n'empêche pas de terminer l'application par un séchage au casque, un brushing ou passage au fer plat ou à friser.

L'invention a également pour objet l'utilisation des compositions décrites ci-dessus pour le traitement cosmétique des matières kératiniques, et notamment des fibres kératiniques telles que les cheveux. Les compositions peuvent notamment être utilisées pour le renforcement des matières kératiniques, et des fibres kératiniques, et notamment pour le renforcement des ongles.

Le procédé peut comprendre une étape d'application sur les matières kératiniques de micro- ou nanoparticules et une étape d'application sur les matières kératiniques d'au moins un monomère électrophile, l'ordre des étapes étant indifférent.

Dans un mode de réalisation particulier, l'application des micro- ou nanoparticules est réalisée avant l'application du ou des monomères électrophiles.

Les exemples qui suivent sont destinés à illustrer l'invention, sans toutefois présenter un caractère limitatif.

Des essais ont été réalisés en utilisant les composés suivants :
- monomère : 2-cyanoacrylate de n-octyle, commercialisé sous la dénomination RITE LOK CON895 par la société CHEMENCE.
- particules 1 : poudre de polytétrafluoroéthylène en dispersion aqueuse à 50% (taille: 900 nm) protege (parabens) commercialisée sous le nom Fluoropure ultrafine 50CW par Shamrock technologies.
- particules 2 : microsphères à enveloppe :chlorure de vinylidene/acrylonitrile/methacrylate de methyle non expansees (10-16 microns) commercialisé sous le nom Expancel 820 DU 40.
- milieu cosmétiquement acceptable : 50% mélange poly dimethylsiloxane alpha-omega dihydroxyle / cyclopenta dimethylsiloxane (14.7/85.3) commercialisé par Dow Corning sous le nom DC 1501 Fluid, avec 50% de cyclopentadimethylsiloxane commercialisé par Dow Corning sous le nom DC 245 Fluid

### Exemple 1

Une dispersion aqueuse est préparée avec 10% de particules 1. 0,5g de cette solution aqueuse est appliqué sur 1g d'une mèche de cheveux naturels propres et secs de hauteur de ton égale à 4 ce qui correspond à une nuance naturelle châtain selon la classification des nuances naturelles décrite dans "Science des Traitements Capillaires" de C. ZVIAK, Ed. Masson 1988, p. 278.

La mèche est ensuite séchée au casque, puis humidifiée avec 0,5g d'eau. Sur cette mèche humidifiée, on applique 0,5g d'une composition contenant le milieu cosmétiquement acceptable décrit ci-dessus et 10% en poids de monomère cyanoacrylate.

Après 10 minutes de pose, la mèche est séchée pendant 2 minutes au sèche-cheveux.

La mèche obtenue présente un volume plus important qu'une mèche non traitée et qu'une mèche qui serait traitée de manière identique mais sans incorporation de particules.

### Exemple 2

Une dispersion aqueuse est préparée avec 10% de particules 1. 0.5g de cette solution aqueuse est appliqué sur 1g d'une mèche de cheveux naturels propres et secs de hauteur de ton égale à 4 ce qui correspond à une nuance naturelle châtain selon la classification des nuances naturelles décrite dans "Science des Traitements Capillaires" de C. ZVIAK, Ed. Masson 1988, p. 278. Sur cette mèche, on applique ensuite 0,5g d'une composition contenant le milieu cosmétiquement acceptable décrit ci dessus et 10% en poids de monomère cyanoacrylate.

Après 10 minutes de pose, la mèche est séchée pendant 2 minutes au sèche-cheveux.

La mèche obtenue présente un volume plus important qu'une mèche non traitée et qu'une mèche qui serait traitée de manière identique mais sans incorporation de particules.

### Exemple 3

On prépare une composition contenant 1 % de particules 2 dans le milieu cosmétiquement acceptable décrit ci dessus. Le monomère cyanoacrylate est ajouté à cette composition de manière à obtenir une concentration finale de 10% en poids de monomère. 0,5g de cette composition est appliqué sur une mèche de cheveux naturels propres et secs de hauteur de ton égale à 4, humidifiée avec 0,5g d'eau.

Après 10 minutes de pose, la mèche est séchée pendant 2 minutes au sèche-cheveux.

On obtient comme précédemment une mèche présentant un volume plus important qu'une mèche non traitée et qu'une mèche qui serait traitée de manière identique mais sans incorporation d'objet.

Dans les exemples 4 à 10, on utilise des microsphères non expansées de copolymère de chlorure de vinylidene/acrylonitrile/méthacrylate de méthyle (10-16 microns) commercialisées sous la référence Expancel 820 DU 40 par la société AKZO NOBEL.

### Exemple 4

La composition suivante est réalisée :

| | |
|---|---|
| DC 1501 Fluid | 45g |
| DC 245 Fluid | 42g |
| Expancel 820 DU 40 | 3g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.

On obtient une mèche présentant un volume plus important qu'une mèche non traitée et qu'une mèche qui serait traitée de manière identique mais sans incorporation d'objet.

### Exemple 5

La composition suivante est réalisée :

| | |
|---|---|
| DC 1501 Fluid | 45g |
| DC 245 Fluid | 41.75g |
| Expancel 820 DU 40 | 3g |
| acide acétique | 0.25g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.

On obtient une mèche présentant un volume plus important qu'une mèche non traitée et qu'une mèche qui serait traitée de manière identique mais sans incorporation d'objet.

### Exemple 6

La composition suivante est réalisée :

| | |
|---|---|
| DC1501 Fluid | 45g |
| DC 245 Fluid | 37g |
| Expancel 820 DU 40 | 3g |
| ethoxyethylcyanoacrylate EO-460 de Tong Shen | 10g |
| acide acétique | 5g |

1.5g de la composition est appliqué sur une mèche de 1g de cheveux propre et humide. Après 15 minutes de pause, la mèche est séchée au sèche-cheveux pendant 2 minutes.

On obtient une mèche présentant un volume plus important qu'une mèche non traitée et qu'une mèche qui serait traitée de manière identique mais sans incorporation d'objet.

### Exemple 7

La composition suivante est réalisée :

| | |
|---|---|
| DC 1501 Fluid | 45g |
| DC 245 Fluid | 41 g |
| Expancel 820 DU 40 | 3g |
| Butylcyanoacrylate B-60 de TongShen | 10g |
| acide acétique | 1 g |

1.5g de la composition est appliqué sur une mèche de 1g de cheveux propre et humide. Après 15 minutes de pause, la mèche est séchée au sèche-cheveux pendant 2 minutes.

On obtient une mèche présentant un volume plus important qu'une mèche non traitée et qu'une mèche qui serait traitée de manière identique mais sans incorporation d'objet.

### Exemple 8

La composition suivante est réalisée :

| | |
|---|---|
| DC 1501 Fluid | 45g |
| DC 245 Fluid | 42g |
| Expancel 820 DU 40 | 3g |
| Ethylhexylcyanoacrylate O-60 de Tong Shen | 10g |

0.5g de la composition est appliqué sur une mèche de 1g de cheveux propre et humide. Après 15 minutes de pause, la mèche est séchée au sèche-cheveux pendant 2 minutes.

On obtient une mèche présentant un volume plus important qu'une mèche non traitée et qu'une mèche qui serait traitée de manière identique mais sans incorporation d'objet.

### Exemple 9

La composition suivante est réalisée :

| | |
|---|---|
| DC 1501 Fluid | 45g |
| DC 245 Fluid | 44g |
| Expancel 820 DU 40 | 1g |
| méthylheptylcyanoacrylate de Chemence | 9g |
| Ethylhexylcyanoacrylate O-60 de Tong Sheng | 1g |

0.5g de la composition est appliqué sur une mèche de 1g de cheveux propre et humide. Après 15 minutes de pause, la mèche est séchée au sèche-cheveux pendant 2 minutes.

On obtient une mèche présentant un volume plus important qu'une mèche non traitée et qu'une mèche qui serait traitée de manière identique mais sans incorporation d'objet.

### Exemple 10

La composition suivante est réalisée :

| | |
|---|---|
| DC 1501 Fluid | 45g |
| DC 245 Fluid | 44g |
| Expancel 820 DU 40 | 1 g |
| méthylheptylcyanoacrylate de Chemence | 7g |
| butylcyanoacrylate B-60 de Tong Sheng | 3g |

0.5g de la composition est appliqué sur une mèche de 1g de cheveux propre et humide. Après 15 minutes de pause, la mèche est séchée au sèche-cheveux pendant 2 minutes.

On obtient une mèche présentant un volume plus important qu'une mèche non traitée et qu'une mèche qui serait traitée de manière identique mais sans incorporation d'objet.

### Exemple 11

La composition suivante est réalisée :

| | |
|---|---|
| DC1501 Fluid | 45g |
| DC 245 Fluid | 42g |
| Talc E0326 commercialisé par la société Luzenac | 3g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.

On obtient une mèche présentant un enrobage rémanent à plusieurs shampooings.

### Exemple 12

La composition suivante est réalisée :

| | |
|---|---|
| DC 1501 Fluid | 45g |
| DC 245 Fluid Ultrafine Zinc Oxide - 350 | 42g |
| (commercialisé par la société Sumitomo) | 3g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.

On obtient une mèche présentant un enrobage rémanent à plusieurs shampooings.

## Revendications

1. Utilisation pour le traitement des matières kératiniques, et notamment des fibres kératiniques telles que les cheveux, d'une composition comprenant, dans un milieu cosmétiquement acceptable, au moins un monomère électrophile et des micro- ou nanoparticules.

2. Utilisation selon la revendication 1, **caractérisée en ce que** le ou les monomères électrophiles répondent à la formule : 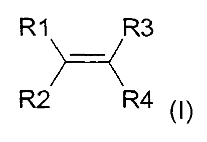 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, - S(R)₂⁺, -SH₂⁺, -NH₃⁺, -NO₂, -SO₂R -C≡N, -COOH, -COOR, -COSR, - CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁- C₄, les groupements aryle et aryloxy,
R désigne un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R' désignant un radical alkyle en C₁-C₁₀.

3. Utilisation selon la revendication 2, **caractérisée en ce que** le ou les monomères électrophiles sont choisis parmi les composés de formule : 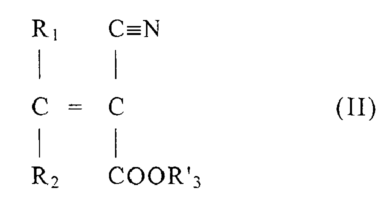 R₁ et R₂ sont tels que définis dans la revendication 3,
R'₃ désigne un atome d'hydrogène ou un radical R tel que défini dans la revendication 2.

4. Utilisation selon la revendication 3, **caractérisée en ce que** le ou les monomères sont choisis parmi les 2-cyanoacrylates de polyfluoroalkyle en C₁-C₂₀, les cyanoacrylates d'alkyle en (C₁-C₁₀) ou d'(alcoxy en C₁-C₄)(alkyle en C₁-C₁₀).

5. Utilisation selon la revendication 4, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

6. Utilisation selon la revendication 3, **caractérisée en ce que** le ou les monomères électrophiles répondent à la formule (F) : 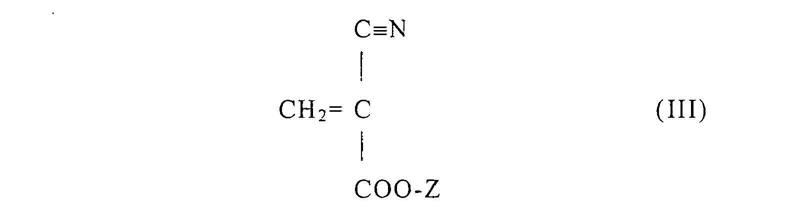 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

7. Utilisation selon l'une quelconque des revendications précédentes, **caractérisée en ce que** le monomère est présent dans la composition à des teneurs comprises entre 0,001 et 80% en poids, de préférence entre 0,1 et 40%, et encore de préférence entre 1 et 20% en poids du poids total de la composition.

8. Utilisation selon l'une quelconque des revendications précédentes, **caractérisée en ce que** les monomères sont fixés de façon covalente sur des supports tels que des polymères, des oligomères ou des dendrimères.

9. Utilisation selon l'une quelconque des revendications précédentes, **caractérisée en ce que** le milieu cosmétiquement acceptable est anhydre.

10. Utilisation selon la revendication 9, **caractérisé en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

11. Utilisation selon l'une quelconque des revendications précédentes, **caractérisée en ce que** la composition comprend des inhibiteurs de polymérisation.

12. Utilisation selon la revendication 11, **caractérisée en ce que** les inhibiteurs sont des inhibiteurs de polymérisation anioniques et/ou radicalaires.

13. Utilisation selon la revendication 11, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

14. Utilisation selon l'une quelconque des revendications 11 à 13, **caractérisée en ce que** les inhibiteurs de polymérisation sont présents en des quantités allant de 10 ppm à 20%, de préférence allant de 10 ppm à 5%, et plus préférentiellement entre 10 ppm et 1% en poids du poids total de la composition.

15. Utilisation selon l'une quelconque des revendications précédentes, **caractérisée en ce que** les macro- ou nanoparticules sont choisies parmi les particules minérales, organiques ou mixtes.

16. Utilisation selon la revendication 15, **caractérisée en ce que** les macro- ou nanoparticules sont choisies parmi les particules métalliques.

17. Utilisation selon la revendication 15, **caractérisée en ce que** les métal des macro- ou nanoparticules métalliques est choisi parmi les métaux alcalins, les métaux alcalinoterreux, les métaux de transition, les métaux des terres rares, et les alliages de ces métaux.

18. Utilisation selon la revendication 17, **caractérisée en ce que** le métal est choisi parmi l'aluminium, le cuivre, le cadmium, le sélénium, l'argent, l'or, l'indium, le fer, le platine, le nickel, le molybdène, le silicium, le titane, le tungstène, l'antimoine, le palladium, le zinc, l'étain, et les alliages de ces métaux.

19. Utilisation selon la revendication 18, **caractérisée en ce que** le métal est choisi parmi l'or, l'argent, le palladium, le platine, le cadmium, le sélénium et les alliages de ces métaux.

20. Utilisation selon la revendication 15, **caractérisée en ce que** les particules inorganiques sont choisies parmi les oxydes, les carbures, nitrures, borures, sulfures et hydroxydes, les sels inorganiques.

21. Utilisation selon la revendication 15, **caractérisée en ce que** les particules minérales sont choisies parmi les argiles, les silicates, l'alumine, la silice, le kaolin, l'hydroxyapatite.

22. Utilisation selon la revendication 21, **caractérisée en ce que** les particules minérales sont choisies parmi les microbilles de silice enrobées de polyméthylhydrogénosiloxane.

23. Utilisation selon la revendication 15, **caractérisée en ce que** les particules organiques sont choisies parmi les poudres de Nylon, les poudres de polyéthylène, les poudres de poly-β-alanine, les poudres polyfluorées, les poudres de copolymère acrylique, les poudres de polystyrène, les poudres de polyester, les microsphères expansées en matériau thermoplastique, les microbilles de résine de silicone, les savons métalliques dérivés d'acides organiques carboxyliques ayant de 8 à 22 atomes de carbone, de préférence de 12 à 18 atomes de carbone, les poudres de polymères hydrophiles, qui sont d'origine synthétique, les polyamides acryliques, les polyuréthanes insolubles, les microsphères poreuses de cellulose.

24. Utilisation selon l'une quelconque des revendications 1 à 15, **caractérisée en ce que** les macro- ou nanoparticules sont sous forme de nanotubes.

25. Utilisation selon la revendication 24, **caractérisée en ce que** les nanotubes sont constitués d'au moins un élément appartenant aux groupes IIA, IIIA, IVA, VA, VIII, IB, IIB, IIIB, VIB et VIIB de la classification périodique des éléments.

26. Utilisation selon la revendication 25, **caractérisée en ce que** les nanotubes sont constitués d'au mois un élément appartenant au groupe IVA de la classification périodique des éléments.

27. Utilisation selon la revendication 26, **caractérisé en ce que** les nanotubes sont constitués de carbone.

28. Utilisation selon l'une quelconque des revendications 1 à 15, **caractérisée en ce que** les nanoparticules sont choisies parmi les nanoparticules semiconductrices luminescentes comprenant au moins un métal choisi parmi Zn, Cd, Hg et au moins un métal choisi parmi S, Se et Te.

29. Utilisation selon la revendication 28, **caractérisée en ce que** les nanoparticules comprennent du séléniure de cadmium ou du sulfure de cadmium.

30. Utilisation selon l'une quelconque des revendications 1 à 15, **caractérisée en ce que** les macro- ou nanoparticules sont choisies parmi les composés susceptibles de gonfler sous l'action de la chaleur.

31. Utilisation selon la revendication 30, **caractérisé en ce que** le composé susceptible de gonfler sous l'action de la chaleur est sous la forme de particules thermoexpansibles.

32. Utilisation selon la revendication 31, **caractérisé en ce que** les particules sont des particules creuses comportant une cavité et une enveloppe continue comprenant au moins un polymère choisi parmi les polymères de chlorure de vinylidène/acrylonitrile/méthacrylate de méthyle, les polymères d'acrylonitrile/méthacrylate de méthyle et les homopolymères d'acrylonitrile.

33. Utilisation selon l'une quelconque des revendications précédentes, **caractérisée en ce que** les macro- ou nanoparticules sont présentes dans la composition à des teneurs comprises entre 0,0001 et 30% en poids, de préférence entre 0,001 et 20%, et encore de préférence entre 0,01 et 10% en poids du poids total de la composition.

34. Utilisation selon l'une quelconque des revendications précédentes, **caractérisée en ce que** la composition comprend en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, cationiques, non ioniques ou amphotères, les polymères fixants ou non, les polyols, les protéines, les vitamines, les colorants directs ou d'oxydation, les agents nacrants, les épaississants minéraux ou organiques.

35. Utilisation selon la revendication 34, **caractérisée en ce que** l'agent est encapsulé.

36. Utilisation selon l'une quelconque des revendications précédentes, **caractérisée en ce que** la composition est sous forme de lotion, de spray ou de mousse.

37. Utilisation selon l'une quelconque des revendications précédentes pour le traitement cosmétique des fibres kératiniques telles que les cheveux.

38. Utilisation selon l'une quelconque des revendications 1 à 36 pour le renforcement des matières kératiniques, et notamment des fibres kératiniques.

39. Utilisation selon la revendication 38, pour le renforcement des ongles.

40. Composition cosmétique, **caractérisée en ce qu'**elle comprend, dans un milieu cosmétiquement acceptable, au moins un monomère électrophile défini dans l'une quelconque des revendications 1 à 7 et des micro- ou nanoparticules autre que les particules constituées exclusivement d'or ou d'argent.

41. Composition selon la revendication 40, **caractérisée en ce que** les particules sont choisies parmi les oxydes métalliques, les particules de polymère, les boîtes quantiques, les nanotubes, les nanofibrilles.

42. Composition selon la revendication 40, **caractérisée en ce que** les micro- ou nanoparticules sont non exclusivement métalliques.

43. Procédé de traitement des matières kératiniques, **caractérisé en ce qu'**il comprend une étape d'application sur les matières kératiniques de micro- ou nanoparticules et une étape d'application sur les matières kératiniques d'au moins un monomère électrophile.

44. Procédé selon la revendication 43, **caractérisée en ce que** l'application des micro- ou nanoparticules est réalisée avant l'application du ou des monomères électrophiles.

45. Procédé de traitement des matières kératiniques, **caractérisé en ce qu'**on applique sur les matières kératiniques une composition utilisée dans l'une quelconque des revendications 1 à 36, en présence d'un agent nucléophile.

46. Procédé selon la revendication 45, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻_{;} OH⁻, ArNH₂, NH₃, I⁻, Br⁻, Cl⁻, RCOO⁻, SCN⁻, ROH, RSH, NCO⁻, CN⁻, NO3⁻, ClO₄⁻ et H₂O, Ph représentant le groupe phényle, Ar représentant un groupe aryle et R représentant un groupe aryle en C₁-C₁₀.

47. Procédé selon la revendication 45, **caractérisé en ce que** l'agent nucléophile est l'eau.

48. Procédé selon l'une quelconque des revendications 45 à 47, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

49. Procédé selon l'une quelconque des revendications 45 à 47, **caractérisé en ce que** les matières kératiniques sont pré-imprégnées à l'aide d'un agent nucléophile autre que l'eau.

50. Procédé selon l'une quelconque des revendications 45 à 47, **caractérisé en ce que** les matières kératiniques sont préalablement réduites avant application de la composition.

51. Procédé selon la revendication 50, **caractérisé en ce que** l'agent réducteur est choisi parmi le thiosulfate de sodium anhydre, le métabisulfite de sodium en poudre, la thiourée, le sulfite d'ammonium, l'acide thioglycolique, l'acide tiolactique, le thiolactate d'ammonium, le mono-thioglycolate de glycérol, le thioglycolate d'ammonium, le thioglycérol, l'acide 2,5-dihydroxybenzoique, le di-thioglycolate de diammonium, le thioglycolate de strontium, le thioglycolate de calcium, le formo-sulfoxylate de zinc, le thioglycolate d'isooctyle, la di-cystéine, le thioglycolate de monoéthanolamine.

52. Procédé selon l'une des revendications 45 à 51, **caractérisé en ce que** la composition comprend en outre un polymère choisi parmi le poly(méthacrylate de méthyle) et les copolymères à base de cyanoacrylates.

53. Procédé selon l'une des revendications 45 à 52, **caractérisé en ce que** l'application de la composition est suivie d'un rinçage.

54. Kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable des micro- ou nanoparticules.
